# EUROPEAN PATENT SPECIFICATION

(11) **EP 3 243 503 B1**
(45) Date of publication and mention of the grant of the patent: **27.05.2020**
(21) Application number: 17386018.0
(22) Date of filing: 08.05.2017
(51) Int. Cl.: A61K 9/00, A61K 9/16, A61L 26/00, A61K 35/644

(54) **THERAPEUTIC PRODUCT FOR THE TREATMENT OF PRESSURE ULCERS THROUGH THE CREATION OF A WOUND-HEALING CRUST UPON SPRAYING**
THERAPEUTISCHES PRODUKT ZUR BEHANDLUNG VON DEKUBITALGESCHWÜREN DURCH ERZEUGUNG EINER WUNDHEILENDE KRUSTE BEIM AUFSPRÜHEN
PRODUIT THÉRAPEUTIQUE POUR LE TRAITEMENT D'ESCARRES DE DÉCUBITUS PAR LA FORMATION D'UNE CROÛTE DE CICATRISATION LORS DE LA PULVÉRISATION

(30) Priority: 09.05.2016 GR 20160100217
(43) Date of publication of application: 15.11.2017
(73) Proprietor: Panagiotou, Petros, 65 403 Kavala (GR)
(72) Inventor: Panagiotou, Petros, 65 403 Kavala (GR)
(74) Representative: Samuelides, Emmanuel

(56) References cited:
- WO-A1-2004/050063
- CN-A- 102 988 424
- US-B1- 9 295 700

## Description

The product belongs to the category of medical devices (formulations) that can be purchased and administered without prescription. The product is a pharmaceutical Galenic formulation that is administered externally. The product is a composition to treat pressure ulcers (bed sores). It is related to a process that uses plant-derived zeolite and menthol powder, as well as plant-based alcohol liquors (propolis, red sweet pepper- Capsicum annuum L., hypericum, aloe, rib-grass, calendula and oregano) that have been converted to powder by the addition of an anti-caking agent. The product can be contained in an aerosol device filled with dimethyl ether (DME) aerosol propellant. Once sprayed on the wound, it strongly adheres and produces a stable wound-healing crust that can be thought of as a type of bandage.

The typical patient groups that suffer from pressure ulcers are patients with special needs, advanced age, paraplegic symptoms and generally patients with restricted movement due to accidents and/or illness. Patients in those groups are usually not in a position to provide the necessary attention to successfully treat the pressure ulcers, which can be infected and in need of long-term treatment. Avoiding further infections is paramount, since the continuous or repeated administration of chemical or antibiotic substances may give rise to multi-drug resistant strains that can make treatment even more difficult. Current products to treat wounds have several disadvantages that delay the repair process. These relate to the following issues:
- The application method of the product.
- The isolation and containment of the wound from any external factor and the outside environment.
- The capacity of the product and its active ingredients to attach and remain on the wound.
- The capacity of the product to provide a persistent administration of an adequate dosage of its active ingredients.
- The maintenance of adequate humidity that is necessary for its treatment.
Known products to heal wounds that include propolis are known from US9295700 and CN102988424.
The invention is defined in the independent claim.

An embodiment of the invention is a liquid composition to treat pressure ulcers (bed sores). It is related to a process that uses plant-derived zeolite and menthol powder, as well as plant-based alcohol liquors (propolis, red sweet pepper- Capsicum annuum L., hypericum, aloe, rib-grass, calendula and oregano) that have been converted to powder by the addition of an anti-caking agent. Zeolite is also added to maintain adequate humidity levels on the wound. The product is contained in an aerosol device filled with dimethyl ether (DME) aerosol propellant. Once sprayed on the wound, it strongly adheres and produces a stable wound-healing crust that can be thought as a type of bandage.

In the dry mass of the active ingredients, propolis has 20-30%, Red Sweet Pepper (*Capsicum annuum L*.) 60-68%, *Calendula officinalis2-3%, Aloe vera 2-3%, Hypericum perforatum* 2-3%, *Plantago spp* 2-*3%, Origanum vulgare* 2-3%, Menthol 0.5-1%, Zeolite 1-3%. The final product can be manufactured as a spray, powder or ointment

The current substance offers the kind of operations that were set as a goal from the beginning of the research. At the same time, it does not contain any harmful ingredient, since its primary sources are of herbal nature. Propolis is one of the most efficient antibiotics in terms of having a wide range of antivarial and antifungal characteristics. It combats all bacterium and parasite while it destroys pathogen organisms which have been resistant to antibiotics. It acts as anti-inflammatory and healing substance. Red Sweet Pepper - Capsicum annuum L. has an efficient antiseptic, anti-inflammatory, and healing operation. The latter becomes more efficient in combination with other pharmaceutical plants' antiseptic and healing operations (Hypericum, Mint, Calendula, Rib-grass, Aloe, and Oregano) which we have selected throughout a process of experiments.

The substance that we produced is composition of the following advantages:
- It contains all the drastic substance of the primary sources that we have chosen
- It's use is done through an easy and safe way
- It is sprayed upon the injury and is perfectly adhered because it can be solidified immediately by creating a stable, healing scab, a kind of bandage
- The immediate adherence of the liquid upon then injury as well as its solidification can isolate at a significant level the injury from its surroundings.
- Spraying and creating a bandage upon the injury ensure the preservation of the drastic ingredients which heal the injury without being swept away
- The adhesive nature of the bandage ensures the substance ingredients' maintenance and absorbance
- The inclusion of a mineral, zeolite, which acts as a humidity controller, preserves the necessary humidity while it also holds the toxins
- The bandage created upon the injury can be isolated easily, when it is considered to be necessary, under the use of a natural serum

The injuries caused by bed rest and by constrained bedsore are very painful and persistent, especially when they are not treated promptly. Very often the injuries are re-infected, while the long-term use of chemicals and antibiotics can lead to the creation of extreme bug stems. These observations as well as the circulation of substances whose implementation is of a minor quality , their potential to remain on the injuries and to maintain the necessary humidity without being blown away, have generated the need for a long-term research which resulted in the creation of a new, easy-to-use, and efficient substance.

The primary source of the manufacturing of the substance was propolis (bee - glue), due to its biological competences. Propolis can act as follows:
- Antibacterial
- Antiviral
- Antifungal
- Antihistamine
- Antiparasitic
- Anti-inflammatory
- Painkilling
- Recovering

Propolis alcoholic extract was produced under winery alcohol of 95%. There was a need for a primary source that can reserve the qualities of propolis and can further be saved easily and under safe conditions. For this reason, it was necessary to mix the alcoholic extract of propolis with an anti-caking agent, the magnesium stearate, with a weight equal to the propolis that was infused. This extract was put into a bottle suitable for vaporizing the alcohol. The rest of the material was transformed with a Blender into a thin dust which was then tested upon injuries. This experiment showed that the dust was adhered to the injuries and cannot be removed unless there was use of a natural serum. As a result, propolis's adhesive body chemistry, one of propolis's natural competences, could be used at a temperature over 30 degrees.

Enrichment of the liquid compound of propolis + magnesium stearate + solvent + propellant (DME) was tested in combination with another drastic chemical in the form of dust. This chemical derived from alcoholic mint extract which was mixed with an anti-caking agent (magnesium stearate) with a weight equal to the mint that was infused. While the alcohol was left to be vaporized, the mint dust was put simultaneously with propolis dust in an aerosol system and solvent - propellant (DME) was added.

It was observed that the kind of bandage, which was created after the new compound had been sprayed onto the injury, incorporated the mint extract. The incorporation of additional drastic chemicals of dust into the primary substance was therefore possible. In other words, we were able to add herbal chemicals resulting from alcoholic extracts so as to reinforce the healing competences of the substance.

For its medical preparation, the following drastic chemicals were used:
- Extract from Red Sweet Pepper *(Capsicum annuum L*), including capsaicin of a heating temperature from 0-100 degrees Scoville and humidity up to 10%. Capsaicin is rich in phenols, carotenoids, vitamins, and other antiseptic and healing competences.
- Extract from well-known pharmaceutical plants with antiseptic and healing competences (Calendula officinalis, Hypericum perforatum, Plantago spp, Aloe vera, Origanum vulgare)
- Herbal menthol of analgesic, antibacterial, antiviral competences
- Zeolite , a 100% clean mineral with special natural competences. It is undiluted, controls the humidity of the environment, and keeps the toxins of the injury.
- An anti-caking agent (magnesium stearate)
- A solvent - propellant (DME)

Alcoholic extracts were produced by the dehydrated parts of the pharmaceutical actors under winery alcohol of 95%. The extracts' substances were mixed with an anti-caking agent (magnesium stearate) of a weight equal to each pharmaceutical actor that had been used, and were put into a bottle suitable for the vaporization of the alcohol. The rest of the material was transformed into blender of a very thin dust.

Participation percentages in the overall compound:
1. 20-30% of propolis + magnesium stearate
2. 60-68% of Red Sweet Pepper (*Capsicum annuum L*)
3. 2-3% of *Calendula Officinalis*
4. 2-3% of *Aloe Vera*
5. 2-3% of *Hypericum perforatum*
6. 2-3% *Plantago spp.*
7. 2-3% of *Origanum vulgare*
8. 0,5-1% of Menthole
9. 1-3% of Zeolite

The dust from all drastic chemicals was put into an aerosol device, where solvent - propellant (DME) was added. The liquid compound produced was sprayed upon the injury and it was solidified immediately. The creation of a kind of healing bandage covered and isolated the injury from its environment, while the interior of the injury maintained the adhesive content of the compound. The detraction of the substance from the injury can be achieved with a natural serum.

## Claims

1. A healing substance that is sprayable on bed-rest wounds to create a stable, healing crust-bandage, the substance having a composition of herbal and natural sources and including the following ingredients i) propolis alcoholic extract, ii) extract from dry parts of Red Sweet Pepper, i.e. *Capsicum annuum L,* including capsaicin of a heating temperature from 0 to 100 degrees Scoville and humidity up to 10%, iii) zeolite powder 100% clean as a controller of humidity, iv) magnesium stearate, v) menthol herbal powder, whereby the substance is in powder form.

2. A healing substance according to claim 1, whereby the mixture of propolis extract with the anti-caking agent is 20% to 30% of the total weight of the substance.

3. A healing substance according to claims 1 or 2, whereby the red sweet pepper is 60% to 68% of the total weight of the substance.

4. A healing substance according to any one of claims 1 to 3, comprising extract from dry parts of *Hypericum perforatum, Aloe vera, Calendula officinalis,* and *Oreganum vulgare.*

5. Ointment including a healing substance according to any one of claims 1 to 4.

6. An aerosol system including the healing substance according to any one of claims 1 to 4 within a solvent propellant.

7. An aerosol system according to claim 6, whereby the propellant is dimethyl ether.

## Patentansprüche

1. Heilsubstanz, die auf Bettruhe-Wunden sprühbar ist, um einen stabilen, heilenden Krustenverband zu erzeugen, wobei die Substanz eine Zusammensetzung aus pflanzlichen und natürlichen Quellen aufweist und die folgenden Bestandteile enthält: i) Propolis-Alkohol-Extrakt, ii) Extrakt aus trockenen Teilen von Roter Paprika, dh *Capsicum annuum L,* einschließlich Capsaicin mit einer Heiztemperatur von 0 bis 100 Grad Scoville und einer Luftfeuchtigkeit von bis zu 10%, iii) Zeolithpulver 100% sauber als Feuchtigkeitsregler, iv) Magnesiumstearat, v) Menthol-Kräuterpulver, wobei die Substanz in Pulverform ist.

2. Heilsubstanz nach Anspruch 1, wobei die Mischung von Propolisextrakt mit dem Antibackmittel 20% bis 30% des Gesamtgewichts der Substanz beträgt.

3. Heilsubstanz nach Anspruch 1 oder 2, wobei der roten Paprika 60 bis 68% des Gesamtgewichts der Substanz beträgt.

4. Heilsubstanz nach einem der Ansprüche 1 bis 3, umfassend einen Extrakt aus trockenen Teilen von *Hypericum perforatum, Aloe vera, Calendula officinalis* und *Oreganum vulgare.*

5. Salbe mit einer Heilsubstanz nach einem der Ansprüche 1 bis 4.

6. Aerosolsystem, das die Heilsubstanz nach einem der Ansprüche 1 bis 4 in einem Lösungs- and Treibmittel enthält.

7. Aerosolsystem nach Anspruch 6, wobei das Treibmittel Dimethylether ist.

## Revendications

1. Substance cicatrisante qui peut être pulvérisée sur les plaies de lit pour créer un bandage de croûte stable et cicatrisant, la substance ayant une composition de plantes et de sources naturelles et comprenant les ingrédients suivants i) extrait alcoolique de propolis, ii) extrait de parties sèches de Poivron rouge, c.-à-d. *Capsicum annuum L,* y compris la capsaïcine d'une température de chauffage de 0 à 100 degrés Scoville et de l'humidité jusqu'à 10%, iii) poudre de zéolite 100% propre comme humidité de contrôle, iv) stéarate de magnésium, v) poudre de menthol à base de plantes , la substance étant sous forme de poudre.

2. Substance cicatrisante selon la revendication 1, dans laquelle le mélange d'extrait de propolis avec l'agent anti-agglomérant représente 20% à 30% du poids total de la substance.

3. Substance cicatrisante selon les revendications 1 ou 2, dans laquelle le poivron rouge représente 60% à 68% du poids total de la substance.

4. Substance cicatrisante selon l'une quelconque des revendications 1 à 3, comprenant de l'extrait de parties sèches *d'Hypericum perforatum,* d'*Aloe vera,* de *Calendula officinalis* et d'*Oreganum vulgare.*

5. Pommade comprenant une substance cicatrisante selon l'une quelconque des revendications 1 à 4.

6. Système d'aérosol comprenant la substance cicatrisante selon l'une quelconque des revendications 1 à 4 dans un propulseur solvant.

7. Système d'aérosol selon la revendication 6, dans lequel le propulseur est l'éther diméthylique.
